# EUROPEAN PATENT SPECIFICATION

(11) **EP 0 698 109 B1**
(45) Date of publication and mention of the grant of the patent: **31.07.2002**
(21) Application number: 94916720.9
(22) Date of filing: 10.05.1994
(51) Int. Cl.: C12N 15/86, A61K 48/00, C12N 15/12

(54) **GENE TRANSFER INTO PANCREATIC EPITHELIAL CELLS**
GENTRANSFER ZU DEN PANKREATISCHEN EPITHELZELLEN
TRANSFERT DE GENES DANS LES CELLULES EPITHELIALES PANCREATIQUES

(30) Priority: 10.05.1993 US 59899
(43) Date of publication of application: 28.02.1996
(60) Divisional application: 01129457.6
(73) Proprietor: THE REGENTS OF THE UNIVERSITY OF MICHIGAN, Ann Arbor Michigan 48109-1280 (US)
(72) Inventor: WILSON, James, M., Ann Arbor, MI 48105 (US)
(74) Representative: VOSSIUS & PARTNER
(86) International application number: US9405187
(87) International publication number: WO9426915

(56) References cited:
- EP-A- 0 561 140
- WO-A-92/05273
- WO-A-93/00051
- WO-A-93/00052
- WO-A-93/03769
- US-A- 5 240 846
- NATURE GENETICS vol. 1, no. 5 , August 1992 pages 372 - 378 JAFFE, H.A. ET AL. 'Adenovirus-mediated in vivo gene transfer and expression in normal rat liver'
- CLINICAL RESEARCH vol. 41, no. 2 , April 1993 MAEDA, H. & CRYSTAL, R.G. 'Gene therapy strategy for the pancreatic insufficiency of Cystic Fibrosis adenovirus -mediated transfer of the human pancreatic lipase cDNA' & Joint Meeting of Assoc. Am. Phys., Am. Soc. Clin. Invest. & Am. Fed. Clin. Invest. WASHINGTON, USA, 1 May 1993
- Raper and Matteo, Pancreas Vol. 12(4): 401-410 (1996)

## Description

The present invention relates to methods for selective somatic gene transfer into a patient's pancreatic epithelial cells. The methods of this invention comprise introducing the gene to be transferred, associated with an appropriate transfer vehicle, into the ductal system of the pancreas. More specifically, the invention relates to using these techniques to treat genetic diseases, such as cystic fibrosis, which are characterized by genetic defects in those epithelial cells.

The epithelial cells lining the ducts of the liver and the pancreas (both endocrine and exocrine) are responsible for synthesizing numerous proteins which are important in maintaining proper functioning of those organs and homeostasis in general. Diseases which cause defects in the genes that encode these proteins can cause serious, and sometimes fatal, effects.

For example, cystic fibrosis (CF) is a disease characterized by abnormalities in water and electrolyte transport into and out of cells. The gene responsible for CF, the cystic fibrosis transmembrane conductance regulator gene (CFTR), is known to be defective in the epithelial cells of CF patients. The isolation and sequence of that gene is described in copending application Serial No. 401,609, filed August 31, 1989. While the primary site of damage in CF patients is the lung epithelia, both the pancreatic and bile duct epithelia are also affected [T. F. Boat et al., in The Metabolic Basis of Inherited Disease - 6th Edition , C. R. Scriver et al., eds., McGraw-Hill, New York, pp. 2649-80 (1989)].

Other diseases characterized by genetic defects in liver and pancreatic epithelial cells are gallstones (cholelithiasis), ascending sclerosing cholangitis, primary biliary cirrhosis and diabetes mellitus.

The treatment of the above-described diseases is of great importance. One exciting approach is the use of gene replacement therapy to insert functional copies of defective genes directly into the affected cells.

The idea of gene replacement therapy was born out of the successful transfer of genes into mammalian cells in culture. The best known method of gene transfer is achieved by treating mammalian cells with a coprecipitate of calcium phosphate and the nucleic acid sequence to be transferred. Mammalian cells take up this precipitate via endocytosis and some of those cells can then express the polypeptide encoded by the nucleic acid sequence. Unfortunately, this technique is limited to in vitro cell cultures and does not have much utility in treating a patient in vivo. This is due to the insoluble nature of the nucleic acids and the low efficiency with which the cells of the body take up these precipitates.

Other techniques, such as the use of viruses or liposomes to carry the nucleic acids to the target cells have more applicability in vivo [C. Nicolau et al., Meth. Enzymol., 149, pp. 157-76 (1987)]. But these methods also suffer from shortcomings. Most importantly, neither of these techniques is specific for any particular cell type -- rendering it difficult to deliver the gene of interest to the proper cells via standard routes of administration.

Recently, a method of cell specific gene transfer utilizing nucleic acids conjugated to cell receptor ligands has been described [United States patent 5,166,320]. While this method provides the cell specificity necessary for in vivo gene therapy, it also involves additional costs and manipulations in creating the nucleic acid-ligand conjugates.

Applicant's copending application Serial No. 584,275, filed September 18, 1990, now United States patent 5,240,846, describes a method of utilizing gene therapy to treat cystic fibrosis. The application describes a number of different gene delivery systems and a number of delivery methods, specifically, inhalation, injection and ingestion. The application is specifically directed to treatment of lung epithelia, but briefly refers to treating pancreatic and biliary epithelial cells. However, the application does not demonstrate that any of the described methods for treating lung epithelia would be effective or specific for pancreatic and biliary epithelia.

Accordingly, there is still a need for an inexpensive and relatively easy way of achieving cell-specific gene transfer to the epithelial cells of the liver and pancreas.

The present invention fulfills this need by providing a novel technique for in vivo gene transfer into pancreatic epithelial cells.

Applicant has discovered that administration of the gene to be transferred, when associated with an appropriate transfer vehicle, into the ductal system of the pancreas causes surprisingly selective uptake by the epithelial cells lining the duct. The administration of the gene can be achieved by methods currently used for injecting contrast dyes into those ducts for imaging techniques.

The methods of this invention are effective in treating primary diseases of the pancreas which are characterized by genetic defects in the epithelia of the organ ductal systems. These genetic defects include both failure of the cells to express a sufficient level of polypeptide, as well as the overproduction of a polypeptide. Such diseases include cystic fibrosis, which affects these cells, as well as lung epithelia.

In another embodiment, the invention provides a method of transferring genes into pancreatic islet cells and pancreatic acinar cells. When the concentration of the transfer vehicle associated with the gene to be transferred is sufficiently high, these additional cells, as well as ductal epithelial cells, take up and express the gene. This represents the first method of achieving gene transfer into pancreatic islet and acinar cells.

The methods of this invention advantageously lower the risks associated with gene transfer by being cell-specific and by avoiding contact with the patient's bloodstream. These methods also take advantage of the anatomical constraints offered by using the ductal system of the pancreas, thus avoiding unwanted gene transfer into other organs and other cells. And the methods of this invention offer an additional advantage of allowing excess genetic material and associated transfer vehicle to be delivered immediately into the duodenum and excreted in the stool.

### BRIEF DESCRIPTION OF THE DRAWINGS

Figure 1 is a schematic representation of the structure of pAd.CMV-lacZ.

Figure 2 is a schematic representation of the structure of pAd.CB-CFTR.

Figure 3 depicts the hybridization of a human CFTR-specific DNA probe to XbaI-digested total cellular DNA from both Ad.CB-CFTR-infected and mock-infected cells derived from a pancreatic adenocarcinoma of a patient with CF.

Figure 4 depicts the hybridization of a human CFTR-specific DNA probe to total cellular RNA from both Ad.CB-CFTR-infected and mock-infected cells derived from a pancreatic adenocarcinoma of a patient with CF.

Figure 5, panels A and B, depict the localization of CFTR in Ad.CB-CFTR-infected cells derived from a pancreatic adenocarcinoma of a patient with CF by immunofluorescence using either a non-reactive antibody (panel A) or a CFTR-specific antibody (panel B) and a second, FITC-labelled anti-IgG antibody.

Figure 6, panels A-E depict the distribution of β-galactosidase in a liver section of a rat at various times after infection with a low concentration of either Ad.CB-CFTR or Ad.CMV-lacZ using X-gal cytochemistry.

Figure 7, panels A-D, depict the presence of human or rat CFTR RNA in a liver section of a rat 3 days after infection with a low concentration of either Ad.CB-CFTR or Ad.CMV-lacZ by hybridization to a labelled CFTR-specific probes.

Figure 8, panels A-D depict the distribution of CFTR and cytokeratin-18 in a liver section of a rat 3 days after infection with a low concentration of Ad.CB-CFTR or Ad.CMV-lacZ by double immunodiffusion using antibodies specific for both proteins.

The present invention provides methods for introducing a functional gene into the pancreatic ductal epithelial cells of a patient. The term "functional gene" as used herein, refers to a gene that encodes a polypeptide and which can be expressed by the target cell. The term also includes antisense nucleic acids which are capable of binding to and inhibiting the expression of a polypeptide. The term "target cell" refers to the cell or cell type which takes up the gene of interest.

The methods of this invention comprise the step of introducing a gene into the ductal system of the pancreas. In order to effectuate gene transfer, the gene to be transferred must be associated with a carrier or vehicle capable of transducing the epithelial cells of the organ. The terms "transfer vehicle" and "carrier" refer to any type of structure which is capable of delivering the gene of interest to a target cell.

Many such carriers are known in the art. For example, various viruses that are capable of infecting epithelial cells can be recombinantly manipulated to carry the gene of interest without affecting their infectivity. As used in this application, the terms "infect" and "infectivity" refer only to the ability of a virus to transfer genetic material to a target cell. Those term do not mean that the virus is capable of replication in the target cell. In fact, it is preferable that such viruses are replication defective so that target cells do not suffer the effect of viral replication.

More preferably, the virus employed to carry the gene in the methods of this invention is a recombinant adenovirus. Adenovirus is preferred for its ability to infect non-dividing or slowly dividing cells, such as epithelia. Most preferably, the recombinant adenovirus is a derivative of Ad5, which has the sequences spanning the E1 region deleted and replaced with a promoter, with or without additional enhancer sequences, and the gene to be transferred. Any promoter which will provide constitutive expression of the gene once incorporated into the target cell genome may be employed. Examples of such promoters are Rous sarcoma virus promoters, Maloney virus LTRs, promoters endogenous to the target cell and cytomegalovirus (CMV) promoters. Preferred promoters are the β-actin promoter and the CMV promoters. Example of preferred enhancer sequences which may be employed in these recombinant adenoviruses include those found in the CMV genome, especially those from the immediate early region of the genome, and alpha fetal protein enhancer sequences.

Other viruses that may be used as transfer vehicles in the methods of this invention are replication defective retroviruses. When these replication defective retroviruses are employed, their genomes can be packaged by a helper virus in accordance with well-known techniques. Suitable retroviruses include PLJ, pZip, pWe and pEM, each of which is well known in the art. Suitable helper viruses for packaging genomes include ΨCrip, ΨCre, Ψ2, ΨAm and Adeno-associated viruses.

Gene delivery systems other than viruses can also be employed in the methods of this invention. For example, the gene to be transferred may be packaged in a liposome. When cells are incubated with DNA-encapsidated liposomes, they take up the DNA and express it. To form these liposomes, one mixes the DNA of an expression vector which expresses the gene to be transferred with lipid, such as *N*-[1-(2,3,-dioleyloxy)propyl]*-N,N,N*-trimethylammonium chloride (DOTMA) in a suitable buffer, such as Hepes buffered saline. This causes the spontaneous formation of lipid-DNA complexes (liposomes) which can be employed in the methods of this invention [P. L. Felgner et al., Proc. Natl. Acad. Sci. USA, 84, pp. 7413-17 (1987)].

Another gene delivery system that may be utilized in this invention is DNA-protein complexes. The formation of these complexes is described in United States patent 5,166,320, the disclosure of which is herein incorporated by reference. Specifically, these complexes comprise the gene to be transferred (together with promoter, enhancer sequences and other DNA necessary for expression in the target cell) linked via a suitable polymer, such as polylysine, to a polypeptide ligand for a receptor on the liver or pancreatic epithelial cell surface. This complex is taken up by the epithelial cells via endocytosis after the ligand binds to the cell surface receptor. The DNA is then cleaved from the rest of the complex via intracellular enzymes which cut the polymer linker.

Once the gene to be transferred is associated with a suitable transfer vehicle, it must be introduced into the ductal system of the pancreas. The preferred route is through the pancreatic duct. In the case of the liver as well as in the case of the pancreas, the genetic material may be delivered to the desired ductal system through the bowel. If only one of the two organs is the desired target, the other can be blocked off by ligature at the point where the duct empties into the bowel.

Any medically accepted method for inserting material into the ducts of these organs can be utilized in this invention. Preferably, the technique employed is minimally invasive and employs a retrograde filling of the ducts. One such preferred technique is the endoscopic retrograde cholangiography procedure (ERCP). ERCP is currently employed to visualize the biliary and pancreatic ductal systems by cannulating the common bile duct or pancreatic duct via an endoscope and injecting contrast dye. In the methods of this invention the gene to be transferred and its associated transfer vehicle is substituted for the dye. Other methods for inserting the gene of interest into the target organ include surgical implantation and insertion via a laparoscope.

According to a preferred embodiment, the invention provides a method for treating pancreatic diseases using the technique of somatic gene transfer. Various diseases are known to be associated with genetic defects of the pancreatic epithelia. In addition, certain symptoms of various diseases of the pancreas are manifest by the epithelial cells of this organ. For example, inflammation of the pancreas could be inhibited by the methods of this invention if used to transfer cytokine genes into the epithelia. Examples of some of the other diseases that may be treated by the methods of this invention include ascending sclerosing cholangitis and primary biliary sclerosis.

According to a more preferred embodiment, the disease to be treated by the methods of this invention is CF. CF is a disease that exerts its primary effect on the lung airway epithelia. However, the disease also affects pancreas and liver epithelia. These secondary disease sites are becoming more important as various therapies to treat CF diseased lungs are developed.

CF can lead to cholestasis, jaundice and eventually cirrhosis in the liver and pancreatitis and malabsorption in the pancreas. Current treatment of CF-related pancreas disorders involves enzyme replacement therapy. However, patients still suffer from pancreatitis and associated malnutrition. There is no current treatment for CF-related liver disorders.

The defective gene in CF is the cystic fibrosis transmembrane conductance regulator (CFTR), a purported transmembrane chloride channel. CFTR expression in the liver has been localized to the epithelial cells which line the biliary tract. In the pancreas, the cells that line the ducts of the exocrine pancreas appear to be the source of CFTR expression. Accordingly, the methods of this invention are well suited for treating CF-related pancreas disorders.

A CFTR cDNA is described in copending application Serial No. 584,274 and in F. S. Collins et al., Science, 235, pp. 1046-49 (1987), the disclosures of which are herein incorporated by reference. That cDNA may be incorporated into any of the gene delivery systems described above and then utilized in the methods of this invention. For example, the construction of certain recombinant viral vectors containing the CFTR cDNA is described in the '274 application. Those vectors are useful in the methods of this invention.

Most preferably, the CFTR DNA is incorporated into a derivative of adenovirus Ad5. The construction of this recombinant vector is described in the specific examples, below.

In order that the invention described herein may be more fully understood, the following examples are set forth. It should be understood that these examples are for illustrative purposes only and are not to be construed as limiting this invention in any manner.

### EXAMPLE 1

### Construction Of Recombinant Adenovirus

### I. pAd.CMV-lacZ

### A. Preparation of a CMV Promoter-lacZ Miniqene

Plasmid pUC19 [C. Yanisch-Perron et al., Gene, 33, pp. 103-19 (1985)] was digested with SmaI and an 8 nucleotide NotI linker was then cloned onto the end. This destroyed the SmaI site, while creating two SacII sites on either side of the linker. A 196 base pair fragment containing the polyadenylation signal of SV40 (SV 40 nucleotides 2533-2729) was then purified from an SV40-containing vector [G. MacGregor et al., Somat. Cell Mol. Gen., 13, pp. 253-65 (1987)]. BamHI linkers were added to that fragment, which was then cloned into the single BamHI site of the modified pUC19 vector. The polyadenylation signal was oriented so that transcription that began with a promoter upstream from the NotI site and passed through that restriction site will encounter the SV40 late gene polyadenylation signal. The SV40 early gene polyadenylation signal is in the opposite orientation.

A 180 base pair XhoI-PstI fragment from plasmid pL1 [H. Okayama et al., Mol. Cell Biol., 3, pp. 280-89 (1983)] containing the SV40 late viral protein gene 16s/19s splice donor and accept signals was then cloned into the above vector to provide the appropriate signals. The human cytomegalovirus immediate early gene promoter and enhancer, obtained as a 619 base pair Thai fragment from pCM5029 [M. Boshart et al., Cell, 41, pp. 521-30 (1985)], was cloned into the HincII site of pUC18 and subsequently recovered from that vector as part of a BamHI/HindIII fragment. That fragment was then treated with T4 polymerase and blunt-end ligated into the above-described modified pUC19 vector.

The E. coli β-galactosidase gene was cut out of pC4AUG [G. R. McGregor et al., Somat. Cell Mol. Genet., 13, pp. 253-65 (1987)] with EcoRI and XbaI as a 3530 base pair fragment. NotI linkers were then added to the fragment and the resulting construct cloned into the NotI site of the above-described modified pUC19 vector.

The entire minigene containing the CMV promoter/enhancer, the lacZ gene and the SV40 polyadenylation signal was then excised from the pUC19 vector with SphI. The fragment was treated with Klenow fragment and ligated with BclI linkers.

### B. Preparation of pAdBglII And Ligation Of the Minigene Into That Plasmid

Plasmid pEHX-L3 [E. Falck-Pedersen et al., J. Virol., 63, pp. 532-42 (1989)], which contains sequences from Ad5 spanning map units 0 to 16.1, was digested with EcoRI and BglII to remove a 5.2 kilobase fragment containing the adenovirus sequences from map unit 9.2 to 16.1, as well as the plasmid backbone. The adenovirus sequences spanning 0 to 1 map units and containing the 5' inverted repeat, origin of replication and encapsidation signals were amplified from the original pEHX-L3 vector and given an NheI site at the 5' end, immediately downstream from the EcoRI site, and a BglII site at the 3' end, using PCR. The PCR-amplified fragment was then ligated to the EcoRI/BglII fragment to produce plasmid pAdBglII.

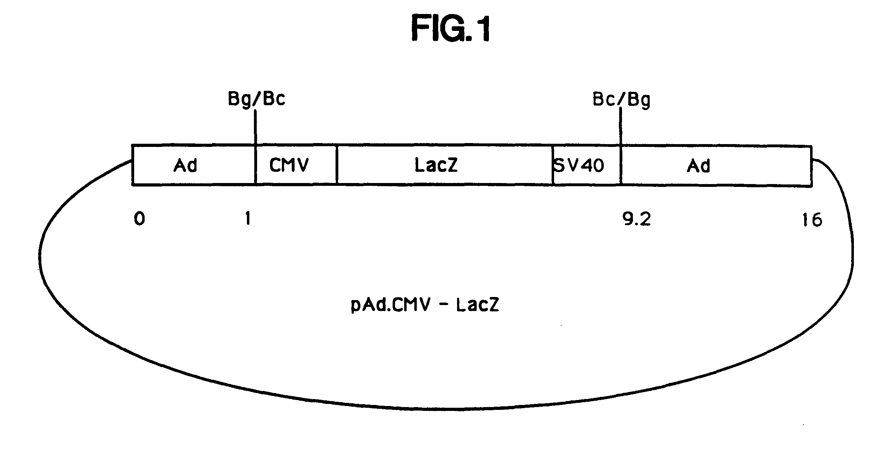

The lacZ-containing minigene prepared as described in part A, above, was then cloned in direct orientation into the pAdBglII vector which had been digested with BglII and treated with calf intestinal phosphatase. A schematic representation of pAd.CMV-lacZ is depicted in Figure 1.

### II. pAd.CB-CFTR

Plasmid pAd.CB-CFTR is derived from pAd.CMV-lacZ. It contains the chicken β-actin promoter, the human CFTR cDNA and a small portion of Mo-MLV retroviral sequences in the place of the CMV promoter and lacZ gene.

### A. Construction of pCMV-BA-CFTR

The vector pBA-CFTR contains an intact 5' LTR of Mo-MLV and additional Mo-MLV viral sequences between the 5' LTR and the internal promoter spanning nucleotides 146 to 624. The plasmid also contains wild-type Mo-MLV sequences from the ClaI site at nucleotide 7674, which was subsequently converted to a BamHI site with synthetic linkers, to the end of the 3' LTR. Sequences containing the viral enhancer elements of the 3' LTR from the PvuII site at nucleotide 7933 to the XbaI site at nucleotide 8111 were deleted. In addition to the above-described sequences, the vector also contains flanking mouse genomic DNA and pBR322 sequences spanning the HindIII site to the EcoRI site.

The β-actin promoter in this vector was derived from a XhoI-MboI fragment of the chicken β-actin gene spanning nucleotides -266 to +1 [T. A. Kost et al., Nucl. Acids Res., 11, pp. 8287-301 (1983)]. The MboI site was subsequently converted to a BamHI site and the modified promoter fragment cloned into the above vector. The human CFTR coding sequences were derived from a 4.6 kb SacI fragment of a CFTR cDNA [J. R. Riordan et al., Science, 245, pp. 1066-73 (1989)] and contained, in addition to the entire CFTR coding region, small amounts of 5' and 3' untranslated regions. The SacI sites were converted to BclI sites and the modified fragment was cloned into the BamHI site of the above vector, immediately following the β-actin promoter.

Enhancer sequences from the immediate early gene of human CMV were obtained by digesting CDM1 [B. Seed et al., Proc. Natl. Acad. Sci. USA, 84, pp. 3365-69 (1987)] with SpeI and PstI, purifying the enhancer sequence-containing fragment and cloning into pUC19. A portion of the enhancer sequence was then excised from that vector with XhoI and NcoI. After purification, the NcoI site was converted to an XhoI site through the addition of synthetic linkers and the modified fragment was cloned into the unique XhoI site located 5' to the β-actin promoter of the above-described vector. The resulting vector was termed pCMV-BA-CFTR.

### B. Replacement of lacZ Minigene In pAD.CMV-lacZ With CFTR Minigene

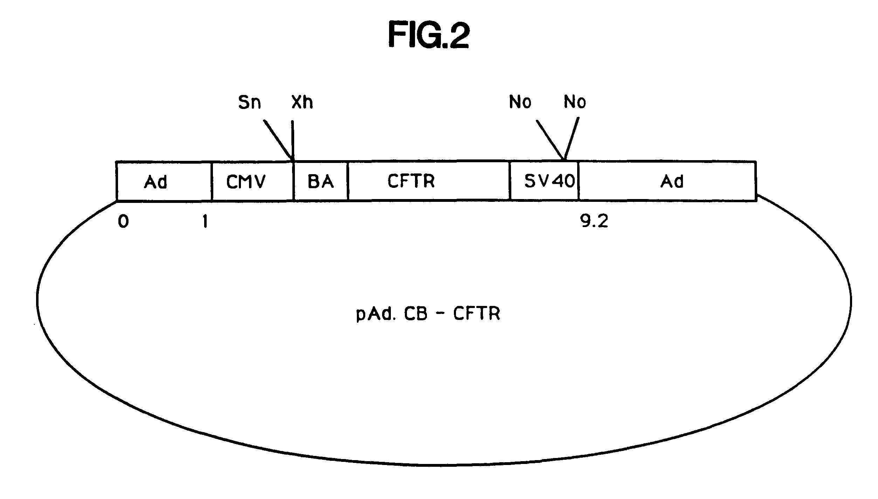

The vector pCMV-BA-CFTR was digested with XhoI and NheI to release a fragment containing the β-actin promoter, the CFTR gene and a small amount of retrovirus-specific sequences and then blunt-ended. Plasmid pAd.CMV-lacZ was cut with SnaBI and NotI to excise the CMV promoter and the lacZ structural gene. The remaining portion of the plasmid, which retained the CMV enhancer and the SV40 polyadenylation signal, was blunt-ended and ligated with the blunt-ended fragment from pCMV-BA-CFTR to form plasmid pAd.CB-CFTR. A schematic representation of pAd.CB-CFTR is depicted in Figure 2.

Plasmids prepared by the processes described above are exemplified by recombinant DNA molecules deposited in the American Type Culture Collection, 12301 Parklawn Drive, Rockville Maryland 20852, USA on May 10, 1993 and identified under the following accession number:
ATCC 75468 - pAd.CB-CFTR.

### III. Generation of Recombinant Ad.CB-CFTR Virus

The vector pAd.CB-CFTR was linearized with NheI and mixed with XbaI digested dl7001 viral genome. The dl7001 virus is an Ad5/Ad2 recombinant virus that has a deletion in the E3 sequences spanning 78.4 to --86 map units. That virus was derived from an Ad5-Ad2-Ad5 recombinant virus made up of the EcoRI fragment of Ad5 spanning 0-76 map units, the EcoRI fragment of Ad2 spanning 76-83 and the EcoRI fragment of Ad5 spanning 83-100 map units. The sequences spanning map units 78.4 to 86 of this Ad5-Ad2-Ad5 recombinant virus were then excised to form dl7001 [Claearas and Wold, Virol., 140, pp. 23-43 (1985)].

I grew 293 cells [F. L. Graham et al., in Methods in Molecular Biology, Vol. 7, E. J. Murray, ed., The Humana Press, Clifton, NJ, pp. 109-28 (1991)] in 150 mm plates containing DMEM supplemented with 10% fetal calf serum, 100 U/ml penicillin and 100 µg/ml streptomycin ("1% pen-strep") until reaching 80% confluency. The cells were then cotransfected with the linearized Ad.CB-CFTR and XbaI digested dl7001. The cells were allowed to grow until plaques formed. Individual plaques were then isolated and amplified in 293 cells. I then isolated viral DNA from individual plaques and analyzed it for the presence of human CFTR DNA via restriction enzyme cleavage and Southern blot analysis.

One of the CFTR-positive plaques was then plaque purified for a second time and the virus therein designated Ad.CB-CFTR. That virus was propagated in 293 cells as follows. Thirty 150 mm plates of 293 cells were grown as described above until reaching 80-90% confluency. The media was removed and the cells were then infected with Ad.CB-CFTR (contained in 10 ml DMEM/1% pen strep) at a m.o.i. of 10 for two hours. I then added 20 ml of DMEM/15% fetal bovine serum/1% pen-strep and continued incubation. At about 36-40 hours post-infection, I harvested the cells by centrifugation and resuspended them in 18 ml of 10 mM Tris-HCl, pH 8.1. The cells were broken open by three rounds of freezing/thawing. The cell debris was then pelleted by centrifugation at 1500 x g for 20 minutes. The supernatant was removed and the pellet was washed once with 10 mM Tris-HCl, pH 8.1. The supernatants were combined and were layered onto 20 ml CsCl step gradients (1.20 g/ml and 1.45 g/ml in 10 mM Tris-HCl, pH 8.1) and centrifuged for 2 hours at 100,000 x g. I then removed the band of viral particles, diluted them in one volume of the Tris buffer and subjected them to a second round of CsCl banding on 8 ml gradients. After centrifugation for 18 hours at 100,000 x g, I recovered the viral particles and stored them in 5 volumes of 10 mM Tris-HCl, pH 8.1, 100 mM NaCl, 0.1% BSA, 50% glycerol. Prior to use I desalted the viral preparation by gel filtration through Sephadex G50 in Hams media.

The final concentration of virus was determined by measuring absorbance at 260 nm. I estimated the titer of the virus via a plaque assay using 293 cells. I also checked for the presence of replication competent virus by infecting HeLa cells at an moi of 10, followed by passaging the cells for 30 days. The presence of replication competent virus is confirmed by observing cytopathic effects in the infected HeLa cells. None of the virus used in the following procedures was replication competent.

### EXAMPLE 2

### Ability of Ad.CB-CFTR To Transform CF Cells

I initially tested the ability of Ad.CB-CFTR to transfer the CFTR gene in the cell line CFPAC, derived from a pancreatic adenocarcinoma of a patient with CF [M. L. Drumm et al., Cell, 62, pp. 1227 (1990)]. I grew these cells at 37°C to confluency in Iscove's modified Delbecco medium (Gibco Laboratories, Grand Island, NY) supplemented with 10% fetal calf serum and 1% pen-strep in 10 cm² plates. I then infected the cells with a Ad.CB-CFTR at an m.o.i. of 1. After 48 hours post-infection the cells were analyzed for gene transfer and expression of CFTR. Analysis was performed on cellular DNA and RNA, as well as using immunocytochemistry to analyze whole cells. Radiolabelled hybridization probes were synthesized from a PCR template derived from the rat CFTR cDNA (nucleotides 1770-2475) described in M. A. Fielder et al, Am. J. Physiol., 262, p. L779 (1992), the disclosure of which is herein incorporated by reference, utilizing the Promega *in vitro* transcription system (Promega Corporation, Pittsburgh, PA) and following the manufacturer's directions. The probes were used in Southern and Northern blot analyses, as well as in in situ hybridization studies.

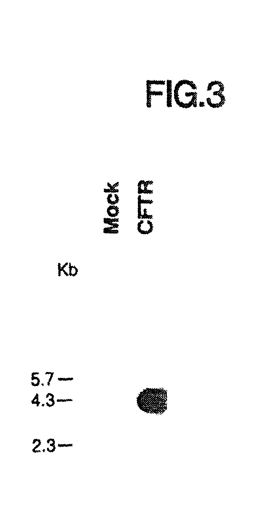

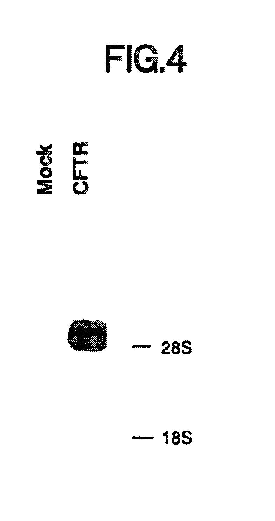

Total cellular DNA from both mock infected and Ad.CB-CFTR infected cells (10 µg) was isolated and digested with XbaI. Southern blots of the DNA derived from the infected cells demonstrated high levels of gene transfer (Figure 3). Total cellular RNA from both mock infected and Ad.CB-CFTR infected cells was isolated, electrophoresed in formaldehyde/agarose gels and transferred to nylon membrane. Northern blots demonstrated an abundant level of CFTR transcripts (Figure 4).

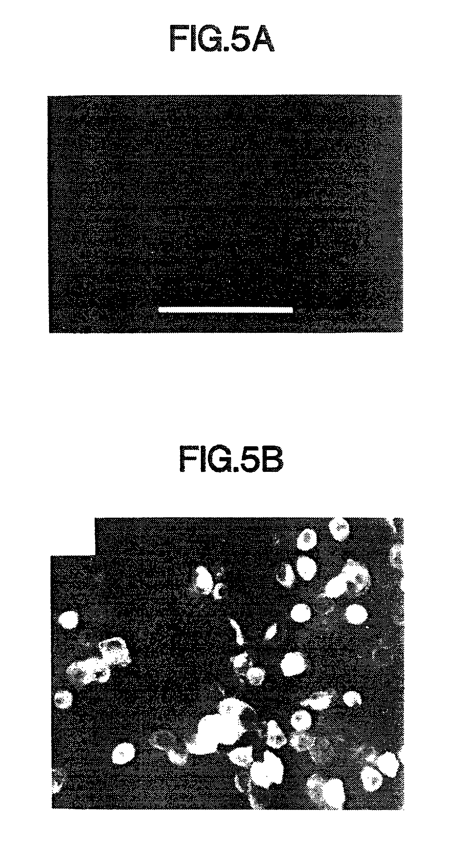

Immunocytochemistry was also performed on the cells to detect CFTR protein. The cells were fixed in methanol at -20°C for 10 minutes and then incubated with 20% normal goat serum in phosphate buffered saline ("GS/PBS") for 30 minutes. The cells were then incubated with 5 µg/ml of a rabbit polyclonal antibody raised against a C-terminal peptide (amino acids 1468-1480) that is conserved in human and rat CFTR [J. A. Cohn et al., Biochem. Biophys. Res. Commun., 181, p. 36 (1991); C. R. Marino et al., J. Clin. Invest., 88, p. 712 (1991); J. A. Cohn et al., Proc. Natl. Acad. Sci. USA, 89, p. 2340 (1992)] in 2% GS/PBS for 50 minutes. Controls included incubation with a non-reactive antibody and pre-incubation of the anti-CFTR peptide antibody with 0.5 mg/ml of CFTR peptide in 2% GS/PBS overnight at 4°C prior to incubation with cells. Following incubation with antibody, the cells were washed three times with 2% GS/PBS for 5 minutes. The antibody was visualized by incubating the cells with a goat anti-rabbit IgG coupled to FITC. The results of this experiment demonstrated that almost all of the cells exposed to the virus expressed detectable levels of CFTR (Figure 5).

The cells were also assayed for the ability to perform cAMP regulated anion conductance -- a characteristic of expression of functional CFTR protein. Specifically, I grew the cells on collagen-coated glass coverslips to confluency under the conditions described above. I then removed the media and replaced it with a hypotonic 1:1 dilution of NaI buffer (130 mM NaI, 4 mM KNO₃, 1 mM Ca(NO₃)₂, 1 mM Mg(NO₃)₂, 1 mM Na₂HPO₄, 10 mM glucose, 20 mM HEPES, pH 7.4) in water. I then added 10 mM (final concentration) of the halide-sensitive fluorophore, 6-methoxy-N-(3-sulfopropyl) quinolinium ("SPQ") and incubated for 12 minutes at 37°C. I removed the SPQ-containing buffer and replaced it with undiluted, isosmotic NaI buffer and incubated the cells for an additional 5 minutes. The coverslips were then transferred to the microscope stage where they were imaged using a 40X oil emersion lens (Nikon CF fluor lens) under light passed through an excitation filter of 370 nm. Emitted fluorescence from the cells was collected by a high resolution image intensifier (Video Scope, Inc, Washington, D.C.) coupled to a video camera. The signal output from the camera was connected to a digital imaging processing board controlled by IMAGE 1/FL software (Universal Imaging, Media, PA).

Almost all of the cells exposed to the virus regained the ability to perform cAMP-regulated anion conductance. This experiment demonstrated that the Ad.CB-CFTR virus was capable of carrying out gene transfer in cells and cure defects in the CFTR gene.

### EXAMPLE 3

### In Vivo Transfection Of Rat Biliary Epithelial Cells

I utilized the Ad.CMV-lacZ virus, described in Example 1, to develop techniques for the in vivo targeting of biliary epithelial cells in rats.

Male Sprague Dawley rats (approx. 200 gms) were anesthetized with isoflurane and their viscera exposed through a midline incision. I then identified the common bile duct and cannulated it with a 27 gauge needle. Various concentrations of virus (1x10¹¹ - 2x10¹² pfu/ml) were suspended in 0.3 ml of phosphate buffered saline and one 0.3 ml aliquot was slowly infused retrograde into each rat. Upon completion of the infusion, I removed the needle and gently applied pressure over the puncture site of the bile duct. The skin and fascia were then closed in one layer with interrupted sutures and the animal was allowed to recover.

As a control, I used the Ad.CB-CFTR virus (1x10¹¹ pfu/ml), described in Example 2 for retrograde infusion.

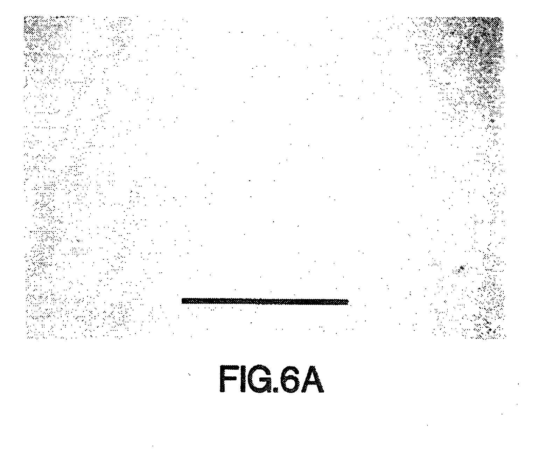

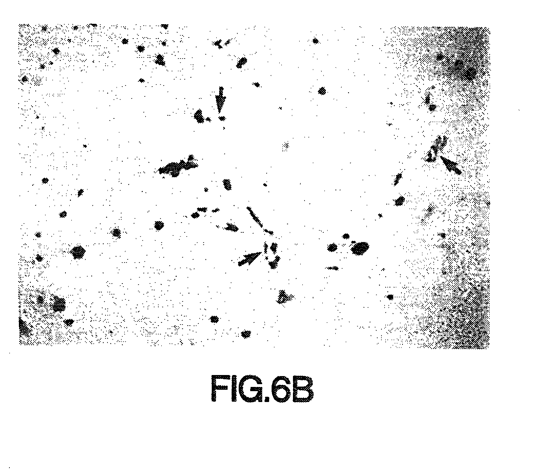

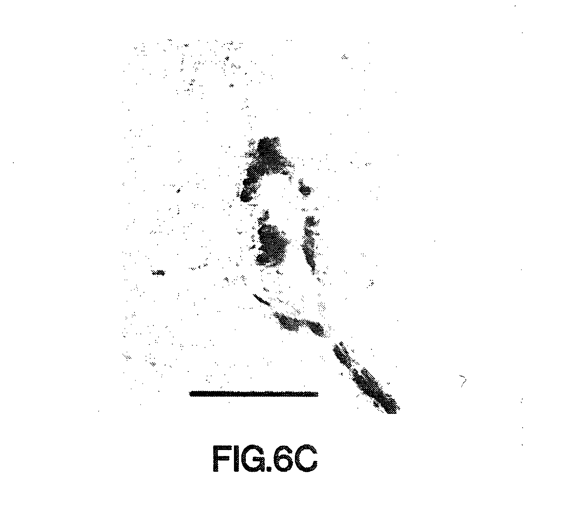

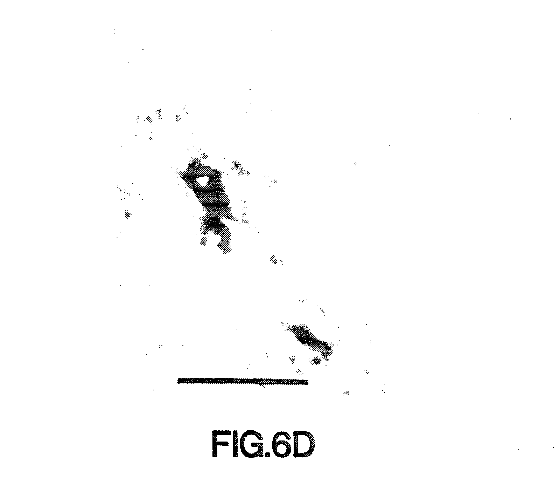

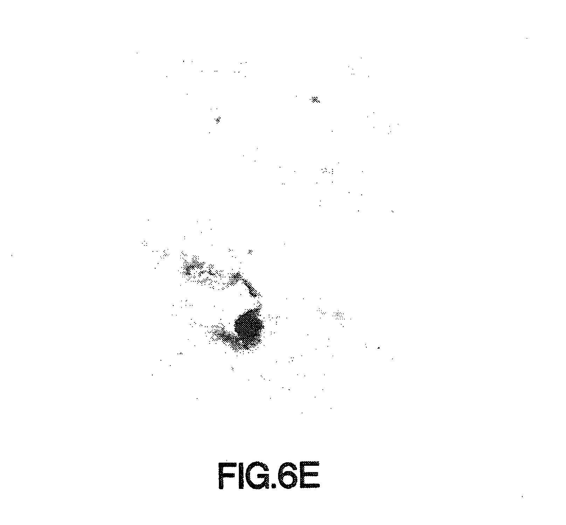

After three days, the animals were euthanized. The liver tissue was then evaluated for lacZ expression using Xgal immunochemistry. Fresh frozen sections (6 µm) were mounted, post fixed in 0.5% glutaraldehyde/PBS for 10 minutes, washed twice with 1 mM MgCl₂/PBS and incubated in Xgal solution (1.6 mg/ml K₃Fe(CN)₆, 2.1 mg/ml K₄Fe(CN)₆•3H₂O, 40 mg/ml Xgal) for 4 hours. Animals infused with the maximum amount of virus (2x10¹² pfu/ml) demonstrated lacZ expression in all of the biliary epithelial cells, as well as over 80% of the hepatocytes. At lower doses of the virus (1x10¹¹ pfu/ml), gene transfer was more selective in that less than 1% of the hepatocytes expressed lacZ, while almost all intrahepatic bile duct epithelial cells continued to express lacZ (Figure 6, panels B and C). Animals infused with Ad.CB-CFTR did not demonstrate lacZ expression (Figure 6, panel A).

In order to evaluate the stability of the expression of this transferred gene, rats receiving the more selective dose of virus were euthanized at 7 and 21 days post-infection. By seven days, expression in hepatocytes and epithelia of the large bile ducts was markedly diminished (Figure 6, panel D). However, lacZ expression in the epithelia of the small biliary ducts remained consistent throughout the 21 days (Figure 6, panel E).

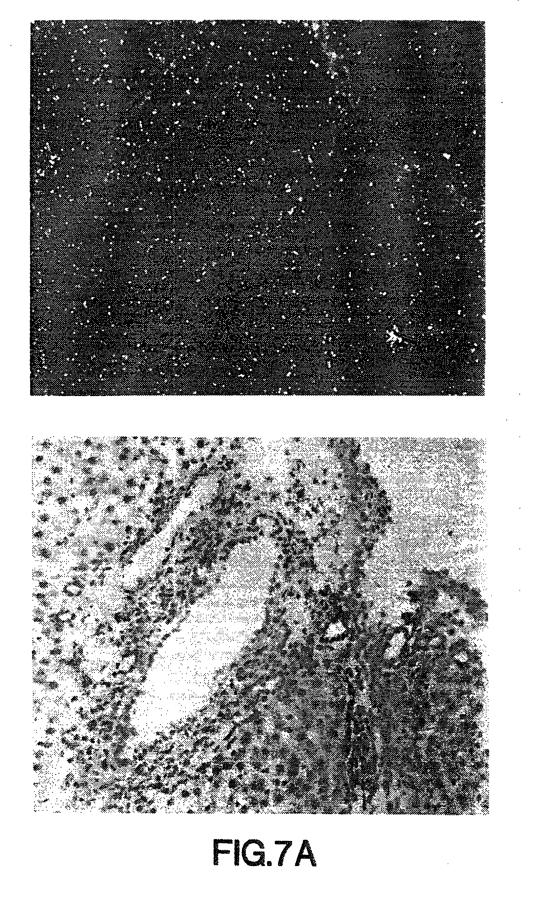

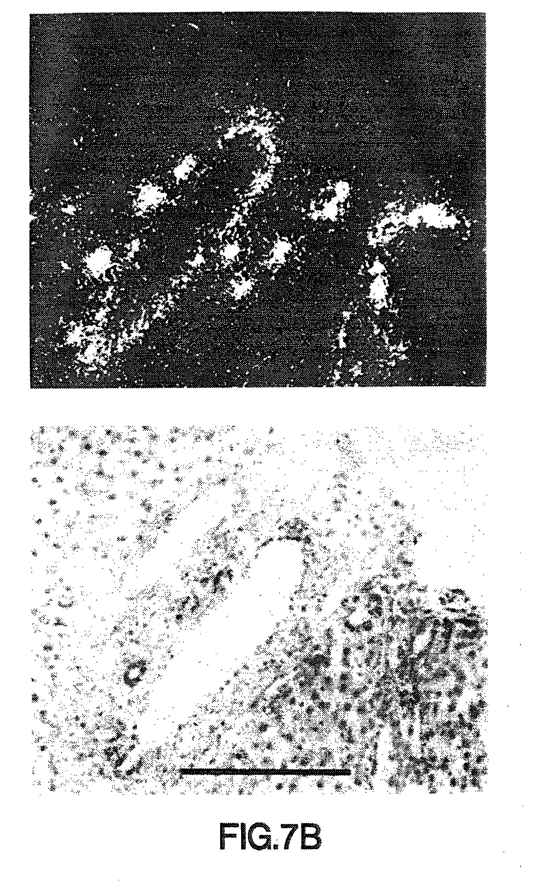

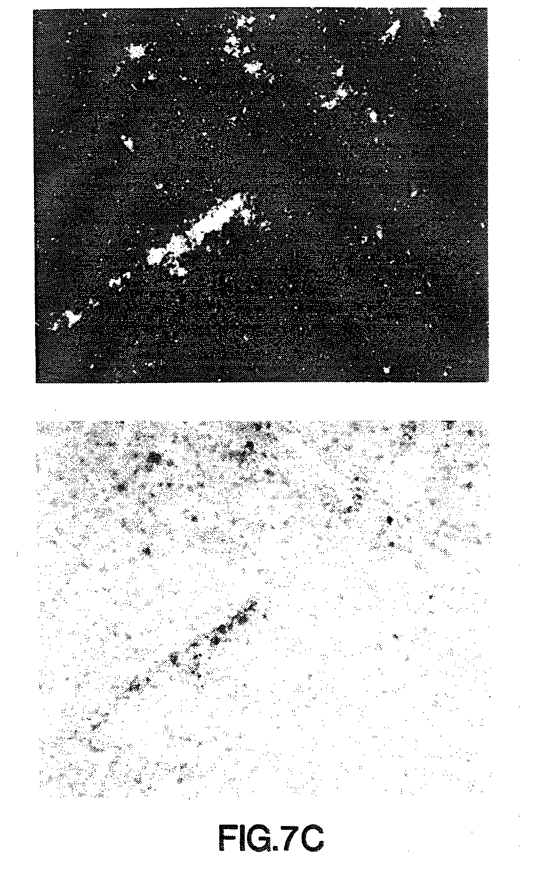

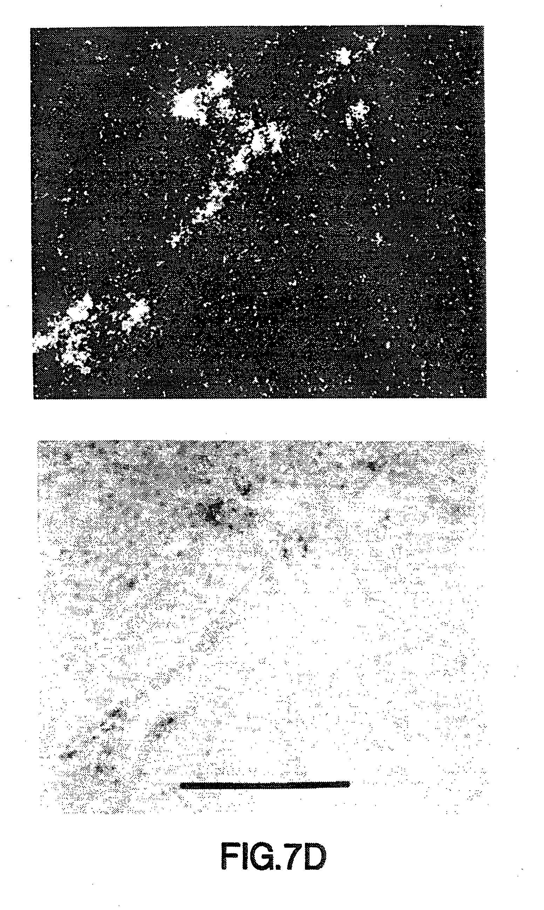

The same types of experiments were repeated assaying for CFTR RNA using rat or human CFTR-specific probes. I used 0.3 ml of 1x10¹¹ pfu/ml of either Ad.CB-CFTR or Ad.CMV-lacZ virus to infect rats. Serial sections of liver taken from infected rats was analyzed for the presence of CFTR RNA using in situ hybridization with probes specific for either rat or human CFTR. The biliary epithelial cells of Ad.CMV-lacZ infected rats hybridized to the rat CFTR probe (Figure 7, panel A), but not to the human probe (Figure 7, panel B). This demonstrated the specificity of the human probe. Animals infected with the Ad.CB-CFTR virus demonstrated a diffuse distribution of human CFTR RNA throughout the biliary epithelial cells of large and small intrahepatic bile ducts (Figure 7, panel C). That distribution was similar to the distribution of the endogenous rat CFTR RNA (Figure 7, panel D). In addition, the human CFTR transcript was detected in a small number of hepatocytes.

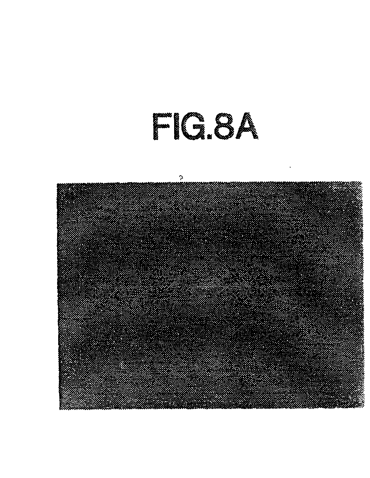

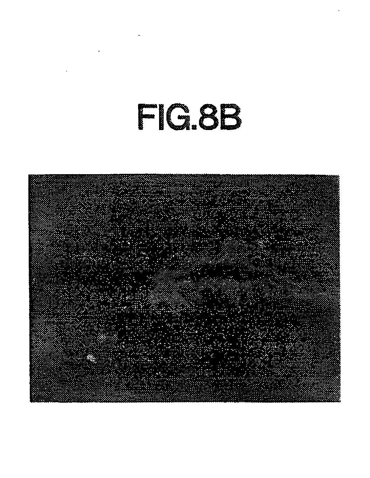

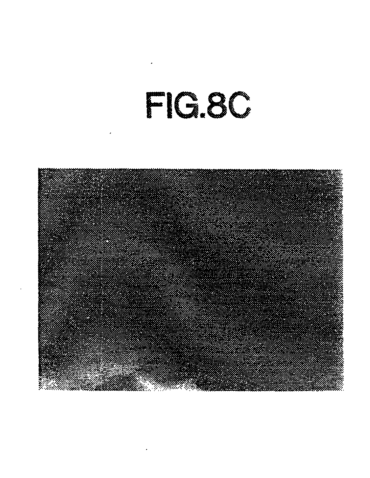

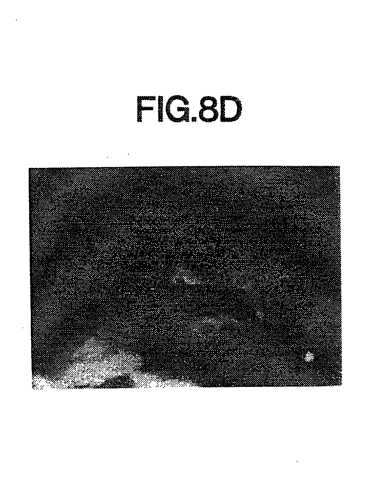

The rat liver sections were also analyzed by double immunodiffusion using antibodies specific for CFTR and for cytokeratin-18, a marker expressed at high levels in biliary epithelial cells. Animals treated with Ad.CB-CFTR demonstrated binding of CFTR antibody to the apical surface of most biliary epithelial cells (Figure 8, panel A). This binding was in far excess of the binding of the antibody to endogenous CFTR protein in Ad.CMV-lacZ infected animals (Figure 8, panel C). The CFTR specific antibody binding detected in Ad.CB-CFTR infected animals localized to the same cells as antibodies to cytokeratin-18 (also demonstrated for Ad.CMV-lacZ infected animals), confirming that the recombinant CFTR is expressed in the proper cell type (Figure 8, panels B and D).

### EXAMPLE 4

### CFTR Gene Transfer Into Biliary And Pancreatic Ductal Epithelia Of Human Patients

Ad.CB-CFTR is used to treat the hepatobiliary and pancreatic aspects of CF. A patient suffering from CF is subjected to endoscopy to visualize the duodenum and locate the common bile duct. Once located, the common bile duct is cannulated. A suitable concentration of virus (approx. 1x10¹¹ pfu/ml) in 50-150 ml PBS is inserted into the common bile duct via endoscopic retrograde cholangiography. Following the procedure, the patient will begin to express CFTR in the biliary epithelial cells.

For treatment of the pancreatic aspects of CF, a similar protocol is followed. A ligature is placed between the liver and pancreatic duct. A needle is then inserted into the bowel to infuse the virus into the pancreatic ducts.

Similar recombinant adenoviruses carrying other genes or cDNA copies thereof are utilized in the same procedure to cure other genetic defects of these epithelial cells. Genetic defects of hepatocytes, pancreatic acinar cells and pancreatic islet cells can also be cured using such recombinant viruses if the viruses are administered into the biliary and pancreatic ducts at a higher concentration.

While I have hereinbefore presented a number of embodiments of this invention, it is apparent that my basic construction can be altered to provide other embodiments which utilize the processes of this invention. Therefore, it will be appreciated that the scope of this invention is to be defined by the claims appended hereto rather than the specific embodiments which have been presented hereinbefore by way of example.

## Claims

1. Use of a functional gene associated with a carrier capable of transferring said gene into pancreatic ductal epithelial cells for the preparation of a pharmaceutical composition for treating a primary hepatic or pancreatic disease in a patient by introducing said functional gene into the pancreatic ductal epithelial cells of a patient via the interlobular ducts of the pancreas.

2. The use according to claim 1, wherein said disease is cystic fibrosis.

3. The use according to claim 2, wherein said functional gene is a gene coding for cystic fibrosis transmembrane conductance regulator (CFTR).

4. The use according to any one of claims 1 to 3, wherein said carrier is a recombinant adenovirus and wherein said functional gene is present in the genome of said adenovirus.

5. The use according to claim 4, wherein said carrier is Ad.CB-CFTR (ATCC 75468).

6. The use according to any one of claims 1 to 5, wherein said functional gene is introduced into the interlobular ducts of the pancreas via a retrograde filling of said ducts.

7. The use according to any one of claims 1 to 6, wherein said retrograde filling is achieved by endoscopic retrograde cholangiography procedure.

8. The use according to claim 1, wherein said gene is also introduced into pancreatic acinar cells and pancreatic islet cells.

## Patentansprüche

1. Verwendung eines funktionellen Gens, das mit einem Träger verbunden ist, der das Gen in Epithelzellen des Pankreasgangs transferieren kann, für die Herstellung eines Arzneimittels zur Behandlung einer primären Leber- oder Pankreaserkrankung bei einem Patienten durch Einführen des funktionellen Gens in die Epithelzellen des Pankreasgangs eines Patienten durch die interlobulären Gänge des Pankreas.

2. Verwendung nach Anspruch 1, wobei die Erkrankung cystische Fibrose ist.

3. Verwendung nach Anspruch 2, wobei das funktionelle Gen CFTR (cystic fibrosis transmembrane conductance regulator) codiert.

4. Verwendung nach einem der Ansprüche 1 bis 3, wobei der Träger ein rekombinantes Adenovirus ist und wobei das funktionelle Gen im Genom des Adenovirus vorhanden ist.

5. Verwendung nach Anspruch 4, wobei der Träger Ad.CB-CFTR (ATCC 75468) ist.

6. Verwendung nach einem der Ansprüche 1 bis 5, wobei das funktionelle Gen in die interlobulären Gänge des Pankreas durch retrogrades Füllen der Gänge eingeführt wird.

7. Verwendung nach einem der Ansprüche 1 bis 6, wobei das retrograde Füllen durch ein endoskopisch-retrogrades Cholangiographieverfahren erfolgt.

8. Verwendung nach Anspruch 1, wobei das Gen auch in die Pankreas-Acinuszellen und Pankreas-Inselzellen eingeführt wird.

## Revendications

1. Utilisation d'un gène fonctionnel associé à un véhicule capable de transférer ledit gène dans des cellules épithéliales du canal pancréatique pour la préparation d'une composition pharmaceutique destinée au traitement d'une maladie hépatique ou pancréatique primaire chez un patient en introduisant ledit gène fonctionnel dans les cellules épithéliales du canal pancréatique d'un patient par les canaux interlobulaires du pancréas.

2. Utilisation selon la revendication 1, dans laquelle ladite maladie est une fibrose kystique.

3. Utilisation selon la revendication 2, dans laquelle ledit gène fonctionnel est un gène codant la protéine transmembranaire CFTR (cystic fibrosis transmembrane conductance regulator).

4. Utilisation selon l'une quelconque des revendications 1 à 3, dans laquelle ledit véhicule est un adénovirus recombinant et dans laquelle ledit gène fonctionnel est présent dans le génome dudit adénovirus.

5. Utilisation selon la revendication 4, dans laquelle ledit véhicule est Ad.CB-CFTR (ATCC 75468).

6. Utilisation selon l'une quelconque des revendications 1 à 5, dans laquelle ledit gène fonctionnel est introduit dans les canaux interlobulaires du pancréas par un remplissage rétrograde desdits canaux.

7. Utilisation selon l'une quelconque des revendications 1 à 6, dans laquelle ledit remplissage rétrograde est effectué par une procédure de cholangiographie endoscopique rétrograde.

8. Utilisation selon la revendication 1, dans laquelle ledit gène est aussi introduit dans les cellules acineuses pancréatiques et les cellules des îlots pancréatiques.
